# EUROPEAN PATENT SPECIFICATION

(11) **EP 0 284 365 B1**
(45) Date of publication and mention of the grant of the patent: **08.06.1994**
(21) Application number: 88302548.8
(22) Date of filing: 23.03.1988
(51) Int. Cl.: A61M 1/00

(54) **Aspirator sleeve**
Ansatz für Sauger
Embout d'aspirateur

(30) Priority: 23.03.1987 US 28788
(43) Date of publication of application: 28.09.1988
(73) Proprietor: Yarger, Richard J., Yakima Washington 98908 (US)
(72) Inventor: Yarger, Richard J., Yakima Washington 98908 (US)
(74) Representative: Bizley, Richard Edward

(56) References cited:
- FR-A- 2 240 026
- GB-A- 1 187 111
- GB-A- 1 209 700
- US-A- 3 771 527

## Description

This invention relates generally to surgical aspirators and more particularly to a releasable sleeve for the tip portion of such aspirators that tends to prevent clogging during use.

Whenever parts of a body are surgically invaded the trauma involved tends to cause the accumulation of body fluids from the tissue itself or from various body fluid systems. The surgical invasion itself normally also creates debris of various sorts, and particularly severed portions of tissue and irrigation fluids, which normally must be removed from the surgical site. Aspirators of many and various types have heretofore become known to remove this material.

In their early history, aspirators generally were formed of elongate metal tubes with a smaller input orifice at a first end and a larger connector orifice at the second end to releasably communicate with an aspirator system providing a waste reservoir and source of vacuum. In use such aspirators were found neither particularly convenient nor reliable as they commonly plugged either from disconnected tissue or other debris in the surgical site or from tissue in place with which they came into contact. With such devices, if the vacuum produced by an aspirator system were too strong, the tip might even damage tissue in place which it contacted, making use not only inconvenient but also requiring that the apparatus be operated with substantial care.

As time progressed and these problems were more fully realised, various solutions were presented.

A surgical drain catheter is described in GB-A-1209700 which includes a generally circular cylindrical cannula which is substantially enclosed in the cylindrical tubular casing. The cannula has a set of oppositely spaced and axially elongate apertures in its distal end radiating outwards. The distal end of the casing also has a plurality of respective oppositely disposed axially elongate apertures, which are oriented generally at right angles to those of the cannula. The extreme distal end of both the cannula and the casing is also provided with an opening.

FR-A-2240026 also discloses a surgical drain. This drain comprises an elongate tubular body having a plurality of spaced aspirator orifices through the wall thereof and can be closed or left open at its distal end.

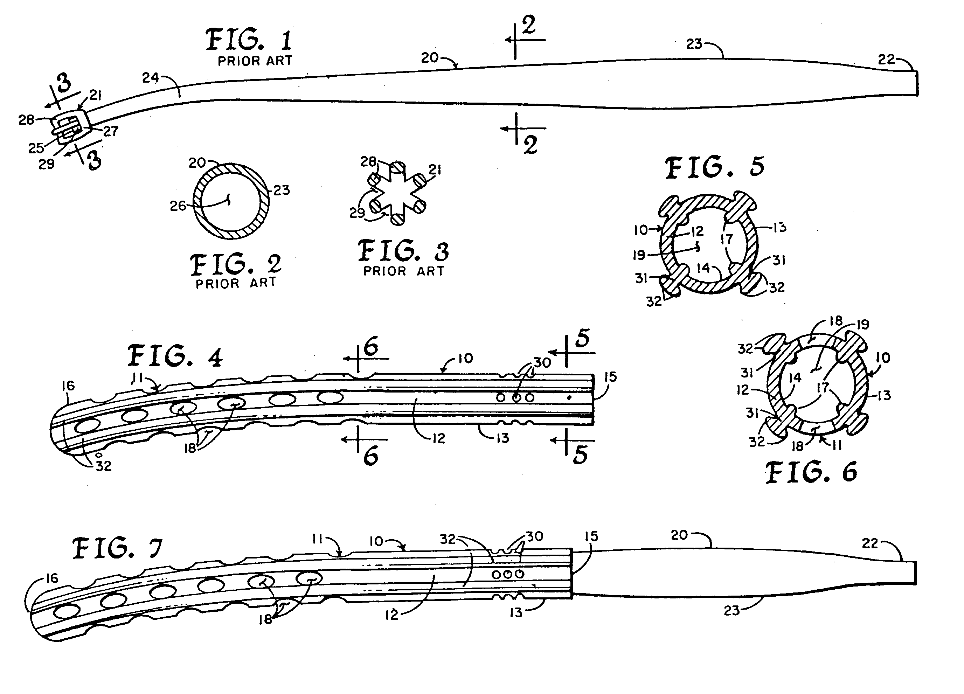

Another solution to the aforementioned problems was proposed in the form of some sort of a guard about the tip or input portion of the aspirator tube. As the tip guard evolved, the straight cylindrical form of the aspirator tube changed to assume a more curvilinear configuration with a medial bulbous enlargement, that served both as a handle structure and a vacuum reservoir, and with a diametrically smaller and generally tapering input portion that was curved, especially near its tip, for easier manipulation and use. A typical aspirator of this type as commonly used in modern day surgery is illustrated in Figure 1 of the drawings associated herewith. In general in the present day such aspirators are designed for single use and are commonly formed of polymeric material because of its transparency and low cost.

Tip guards are commonly used with such aspirator tubes but such guards have not become particularly standardised and heretofore have taken many and various forms. All such tip guards, however, have generally had the same essential characteristics of being quite small in both axial and radial dimension, being irreleasably positionable on or in the immediate vicinity of the tip, and having a plurality of orifices defined therein so that if one orifice plugs others may remain operative. By reason of these characteristics, the guarded aspirator tip is more functional than an unguarded tip, but it is not too much better, as the guarded tip still tends to plug and clog.

Orifices defined in tip guards generally are of a size as large or larger than the associated aspirator tip orifice, so if tissue or debris be presented to them it may commonly pass therethrough to plug the input orifice of the aspirator tube. More importantly when an aspirator with this type of tip guard be used in a surgical site adjacent a surface of soft tissue that tissue may move and be deformed, both by the manipulation of the aspirator and by its vacuum, so that commonly such tissue may plug all of the orifices defined in an aspirator guard. This tendency is roughly proportionable to the external size of the tip guard.

According to the invention there is provided a sleeve in combination with an elongate surgical aspirator tube having either a straight or a curved profile, the aspirator tube having an enlarged medial portion, a forward portion extending forwardly of the medial portion, a tip in the end of the forward portion opposite the medial portion and with the forward portion being diametrically smaller than the enlarged medial portion; said sleeve comprising an elongate, tubular body defining an internal channel having an open, rearward aspirator end and a forward tip end; said tubular body being deformable to slidably engage over and assume the profile of the forward portion of the surgical aspirator tube; said body defining plural, spaced orifices at least in the forward tip portion, at a spaced distance rearwardly from the tip end, to communicate through the tubular body to allow passage of material to be aspirated into the internal channel defined by the tubular body; characterised in that the tip end of the sleeve is closed and the channel of said tubular body is configured to releasably receive the forward portion of the aspirator tube therein and in which the forward portion and tip of the aspirator tube are securely but releasably received therein; and in that the open, rearward end of the tubular body has a resilient expandable section that is capable of significantly expanding in size to frictionally engage around the enlarged medial portion of the aspirator tube.

The present invention seeks to prevent plugging of the aspirator orifices by providing an auxiliary closed-end sleeve to fit about both aspirator and tip guard. The sleeve takes the configuration of a cylindrical tube and is formed of resiliently deformable material so that it will deform to accommodate the curvilinear shape of the tip of present day aspirators. The tube defines plural, spaced, relatively small orifices over a substantial portion of its area and is dimensioned so that it is positionally maintained by frictional engagement on the aspirator tube.

Preferably the inner surface of the sleeve defines a plurality of inwardly projecting elements to positionally maintain the aspirator tube being serviced in a medial position within the channel defined by the sleeve.

Preferably the surface of the sleeve has a plurality of outwardly projecting ribs to aid in spacing the orifices defined in the sleeve from tissue defining a surgical site.

The elongate axially aligned ribs are provided on the external surface of my guard to further aid in preventing clogging of orifices, especially as by tissue in place surrounding a surgical site. With such size and configuration it becomes quite unlikely that all of the orifices in the sleeve may be plugged at one time, either by debris at the surgical site or by adjacent soft tissue. My sleeve is especially useful in the gross cleaning of surgical sites and for use in larger sites. If a smaller working area be required than is allowed by my sleeve, the sleeve may readily be removed and either the aspirator tube with tip guard, or if necessary without tip guard, used independently. The peripheral surface of the sleeve, wherein orifices are defined, is of substantially greater area than are the peripheral surfaces of tip guards heretofore commonly used on surgical asprirators, differing normally by a factor of fifty or more.

Preferably the sleeve is formed from a resiliently deformable material.

In a preferred embodiment of the invention the size of the internal channel of the tubular body is sufficiently larger than the exterior of the forward portion of the aspirator tube to enable the material passing into the internal channel through the orifices to flow through the internal channel between the interior of the tubular body and the exterior of the aspirator tube toward the forward tip of the aspirator tube.

Preferably the orifices are arrayed in radially spaced axially extending lines and the inner and outer spacing ribs are radially coincident and extending between the lines of spaced orifices.

The sleeve, when used in association with a tip guard, provides a double orifice system through which any matter must pass before it might arrive at an aspirator tube tip to plug that tip and obviously the probability of such debris passing through both orifice systems is less than the probability of its passing through only one. This action is further enhanced by the internal ribs defined on the interior surface of my sleeve which maintain it at a spaced distance outwardly from the periphery of an associated aspirator tube. This structure provides auxiliary orifices for the entry of air at the junction of the inner end of my sleeve with an aspirator tube to make it very nearly impossible to plug all of the sleeve's orifices at one time under any conditions, and also requires that there will always be space between the aspirator and my sleeve wherein fluids may collect and debris may remain without moving to or through either the tip guard or aspirator tube tip.

In a preferred embodiment of the invention the sleeve is combined with an elongate aspirator tube having an enlarged medial portion, a forward portion extending forwardly of the medial portion, a tip in the end of the forward portion opposite a medial portion and with the forward portion being diametrically smaller than the enlarged medial portion.

A principal object of the invention is to create a sleeve for surgical aspirator tubes having a tip guard that allows the normal functioning of the aspirator but aids in preventing its clogging from either debris or soft tissue adjacent a surgical site.

A further preferred object of the invention is to provide such a sleeve that has ribbed inner and outer surfaces to maintain the sleeve at a spaced distance from the aspirator to be serviced and from soft tissue defining a surgical site, to aid in preventing the blocking of either sleeve or aspirator orifices.

A further object of the invention to provide such a device that has relatively small spaced orifices defined over a substantial part of both the tip and inner portion of its surface to make unlikely the possibility that all orifices may be simultaneously plugged.

A still further object of the invention to provide such a sleeve that is formed of resiliently deformable material, such as a transparent polymer, that may conform to the curvilinear shape of a particular aspirator tube and allow viewing of that tube.

A still further object of my invention to provide such a sleeve that is of new and novel design, of rugged and durable nature, of simple and economic manufacture and one otherwise well adapted to the uses and purposes for which it is intended.

In the accompanying drawings which form a part hereof and wherein like numbers of reference refer to similar parts throughout:
Figure 1 is an orthographic surface view of a typical curvilinear aspirator tube and tip guard heretofore known in the prior art and commonly used in the modern day surgical practice.
Figure 2 is a somewhat enlarged transverse cross-sectional view through the medial portion of the aspirator tube of Figure 1, taken on the line 2-2 thereon in the direction indicated by the arrows.
Figure 3 is a somewhat enlarged transverse cross-sectional view through the tip guard structure of the aspirator tube of Figure 1, taken on the line 3-3 thereon in the direction indicated by the arrows.
Figure 4 is an orthographic side view of the sleeve of my invention showing its various parts, their configuration, and relationship.
Figure 5 is a somewhat enlarged transverse cross-sectional view through the open end portion of the sleeve of Figure 4, taken on the line 5-5 thereon in the direction indicated by the arrows.
Figure 6 is a somewhat enlarged transverse cross-sectional view through the orifice structure of the sleeve of Figure 4, taken on the line 6-6 thereon in the direction indicated by the arrows.
Figure 7 is an orthographic side view of the sleeve of my invention in operative position on a prior art aspirator tube and guard such as illustrated in Figure 1.

One embodiment of my invention comprises a cylindrical sleeve, having one closed end, to be releasably positioned about the tip portion of a surgical aspirator to aid in preventing clogging. The sleeve is formed of resiliently deformable material that defines plural spaced orifices and ribs on both interior and exterior surfaces to allow entry of fluid and small debris to the aspirator therein but aid in preventing the entry of tissue or larger debris that causes clogging or plugging. The ribs and additional fenstrae maintain aspirator action and prevent its discontinuance by non-dissected tissue's blocking of sleeve orifices.

My invention generally provides sleeve body 10 defining orifices 11.

Body 10 provides cylindrical tube 12 having outer peripheral surface 13 and inner peripheral surface 14 with open aspirator end 15 and enclosed tip 16. The tip is preferably configured substantially as a hemisphere, as illustrated, to provide no sharp edges or corners that might either be physically damaged or might themselves physically damage soft tissues surrounding a surgical site.

Inner surface 14 of body 12 defines plural, elongate, axially extending, inwardly projecting positioning ribs 17, four in the instance illustrated, arrayed with circular symmetry about the inner surface of the body. The number, arrangement, and positioning of these ribs is not particularly critical, though generally it is more convenient if they be at least three in number so arrayed as not to coincide with orifices defined in the tip portion of the body.

Outer surface 13 of the body defines plural spacing ribs 31 again axially aligned and projecting radially outwardly a spaced distance from the body. Generally inner and outer ribs are radially aligned with each other in cooperating pairs to position them between orifices or fenstrae. The radially outer parts of the outer ribs may provide somewhat bulbously enlarged portions 32 to aid in spacing the body from tissue defining a surgical site, though this feature, if used, may make the formation process more difficult.

The body of my invention is formed of some semi-rigid, resiliently deformable material that is adaptable for use in the medical arts. I prefer a polymeric or resinous plastic material such as is commonly used in surgical apparatus in the present day. With the economics of modern medical practice, the sleeve will normally be provided for one time use in a sterilized container. This, of course, is not necessary to my invention and obviously materials that may be sterilized between uses may be provided. In general though, in general sterilization and recyling for reuse may cost more than the initial cost of the product. My sleeve may be formed by various of the manufacturing processes used for such purposes in the present day. A length of continuous extruded tubing of appropriate cross-section may be cut to appropriate length, with the tip being closed by deformation and joinder. The structure might also be formed by injection molding if this type of formation process be desired.

Positioning ribs 17 preferably, but not necessarily, are formed with rounded inward projections so that a smaller surface area might contact an aspirator tube to be serviced to allow easy placement and removal of my sleeve. Often if any substantial area of a sleeve be in direct frictional contact with an aspirator tube, the two may generate substantial frictional forces and be fairly difficult to move relative to each other.

Orifice structure 11 of my sleeve includes a plurality of spaced holes 18 defined through the walls of body 12. These holes in the instance shown are shaped in the form of ellipses with about 25 degrees of ellipticity. This shape obviously is not necessary but provides an orifice of greater area but lesser dimension in one direction which oftentimes may prohibit the passage of elongate particles that could pass through the orifice except for their orientation relative thereto. There is no required number, size or array of these orifices but the configuration illustrated has been found empirically to be quite functional. As illustrated, holes 18 are arrayed in four axially parallel lines and in the case of ellipses are oriented with longer axes along an axially extending line with spacing substantially a hole length apart. The holes in adjacent lines are so related that they would be staggered relative to each other. The size of the holes is preferably such that the holes may be arrayed between adjacent positioning and spacing ribs defined on the surfaces of body 12. Normally these holes are formed in resiliently flexible plastic by punching with a die aided by an internal arbor, but they may be formed by other of the processes presently known in the plastic manufacturing arts.

Additional orifices or fenstrae 30 may be defined immediately inwardly adjacent aspirator end 15 of the body to assure airflow through the sleeve if other orifices should become plugged. These fenstrae are not essential to my sleeve but do provide more absolute assurance of operability.

The configuration and sizing of my sleeve is somewhat critical. Tubular chamber 19 defined by tubular body 12 should be somewhat larger than the tip portion of an aspirator tube to be serviced but yet should not be larger than the medial bulbous portion of that aspirator tube upon which my tube is to be positioned so that the tube may be frictionally maintained in its rearward or inner part. Normally this will require a plastic tubing of approximately one-half inch external diameter depending upon aspirator size, sleeve tube thickness and depth of inward projection of the positioning ridges. It is not necessary, and in fact not desirable, that the tip guard of an aspirator tube to be serviced extend completely to the end of my sleeve. It is preferable that the tip guard be a spaced distance from a sleeve tip so that the tip portion of the sleeve may tend to form somewhat of a reservoir for collection of fluids to be aspirated. The sizing of the aspirator tube in Figure 1 and of the sleeve in Figure 4 are approximately at full scale.

Having thusly described the structure of my invention, its operation may be understood.

The type of aspirator tube 20 and tip guard 21 with which my invention is particularly designed to function is illustrated in Figure 1. Aspirator tube 20 in general provides a tubular structure of circular cross-section having smaller connecting portion 22 in its rearward part communicating with medial, larger bulbous portion 23, which forms a handle and reservoir to stabilize vacuum in the device, which in turn communicates with forwardly tapering and curved neck portion 24 which terminates in aspiration orifice 25. The thickness of the wall defining aspirator tube 20 is relatively uniform throughout so that chamber 26 defined therein is of shape slightly smaller but substantially similar to that of the external periphery.

Tip guard 21 provides connector ring 27 sized and configured to fastenably engage the outer surface of the aspirator tube immediately inwardly of orifice 25. The connector ring structurally communicates with and supports tip structure 28 defining plural orifices 29 for the passage of material to be aspirated. The tip structure normally is relatively small and generally of a diameter somewhat less than the largest diameter of an associated aspirator tube. The form of aspirator tube and tip guard illustrated is well known and commonly used in modern day surgical arts.

To use my sleeve a device is formed, according to the foregoing specifications, to the configuration illustrated in Figure 4. Tip guard 21 of an aspirator to be serviced is inserted through inner open end 15 of sleeve body 12 and the sleeve thereafter manually moved upon the aspirator tube until its inner aspirator end becomes frictionally engaged with the tip facing portion of bulbous enlargement 23. As the sleeve moves upon the curved tip portion of the aspirator, it will assume generally the same curvilinear shape of the aspirator tube by reason of its resilient nature and interfitting relationship with the aspirator tube. Normally tip guard 21 of an aspirator tube to be serviced will be of a size appropriate to fit within channel 19 defined by my sleeve, and, if so, the tip guard should be left in place on an aspirator tube when used with my invention. If a tip guard is too large to fit within a sleeve cavity, the guard may be removed. Some tip guards are releasably maintained on aspirator tubes and, if this be the case with a particular tip guard, it may be manually removed; if this not be the case, the tip guard may be mechanically severed at its inner end for removal.

When my sleeve is positioned on an aspirator tube as aforesaid, it is to be noted that the inner or aspirator end 15 of the sleeve will contact the aspirator tube by means of the inner portions of positioning ridges 17, so that there will be orifices defined between my sleeve, the aspirator tube, and each of the positioning ridges. By reason of the general operation of the device and the position of these orifices they almost always will be in a position where they are not and cannot be simultaneously blocked, so that if all of the other orifices defined in the sleeve become plugged or blocked the aspirator will still draw air from the ambient atmosphere through the orifices between the tube and the aspirator end of my sleeve.

Once my sleeve is established on an aspirator tube, the combination is used substantially in the same manner as an ordinary aspirator tube without the sleeve. The forward portion of the device is placed in the lower portion of a surgical site and various fluids and debris therein will tend to pass through holes 18 in the forward portion of the sleeve to tip orifice 25 of the aspirator tube being serviced from whence they are exhausted through the aspirator in the ordinary fashion. Depending upon the manner of manipulation of an aspirator using my sleeve, the tip portion of the sleeve may serve as a reservoir for the collection of material to be aspirated, especially as when the tube assumes a somewhat vertical position with tip lowermost. Should orifices of my sleeve become plugged or should the aspirator become plugged, my sleeve may be readily removed, if necessary, by manual manipulation, in reverse of the manner in which it was established, to allow cleaning of its chamber and any of the orifices.

It is further to be noted that my sleeve could be used as an aspirator tube per se if it be interconnected with some source of vacuum at its inner or open aspirator end. This, however, is not its primary purpose and with its multiple orifice structure distributed over a relatively large area, its operation as an aspirator tube per se would be rather gross and not too finely regulatable, if in fact ordinary aspirator mechanism would support its operation.

## Claims

1. A sleeve in combination with an elongate surgical aspirator tube (20) having either a straight or a curved profile, the aspirator tube having an enlarged medial portion (23), a forward portion (24) extending forwardly of the medial portion, a tip in the end of the forward portion opposite the medial portion and with the forward portion being diametrically smaller than the enlarged medial portion; said sleeve comprising an elongate, tubular body (12) defining an internal channel (19) having an open, rearward aspirator end (15) and a forward tip end (16), said tubular body being deformable to slidably engage over and assume the profile of the forward portion of the surgical aspirator tube; said body defining plural, spaced orifices (18) at least in the forward tip portion, at a spaced distance rearwardly from the tip end, to communicate through the tubular body to allow passage of material to be aspirated into the internal channel (19) defined by the tubular body; characterised in that the tip end of the sleeve is closed and the channel of said tubular body is configured to releasably receive the forward portion of the aspirator tube therein and in which the forward portion and tip of the aspirator tube are securely but releasably received therein; and in that the open, rearward end of the tubular body has a resilient expandable section that is capable of significantly expanding in size to frictionally engage around the enlarged medial portion of the aspirator tube.

2. A sleeve and aspirator tube combination as claimed in claim 1 in which the inner surface of the sleeve (14) defines a plurality of inwardly projecting elements (17) to positionally maintain the aspirator tube being serviced in a medial position within the channel (19) defined by the sleeve.

3. A sleeve and aspirator tube combination as claimed in claim 1 or claim 2 in which the outer surface of the sleeve (13) has a plurality of outwardly projecting ribs (31) to aid in spacing the orifices (18) defined in the sleeve from tissue defining a surgical site.

4. A sleeve and aspirator tube combination as claimed in any one of claims 1 to 3 formed from resiliently deformable material.

5. A sleeve and aspirator tube combination as claimed in any one of claims 1 to 4, wherein the size of the internal channel (19) of the tubular body (12) is sufficiently larger than the exterior of the forward portion of the aspirator tube (20) to enable the material passing into the internal channel (19) through the orifices (18) to flow through the internal channel (19) between the interior of the tubular body (12) and the exterior of the aspirator tube (20) toward the forward tip of the aspirator tube (20).

6. A sleeve and aspirator tube combination as claimed in any one of the preceding claims in which the orifices (18) are arrayed in radially spaced, axially extending lines and the inner and outer spacing ribs (17,31) are radially coincident and extending between the lines of spaced orifices.

## Patentansprüche

1. Hülse in Kombination mit einem länglichen chirurgischen Absaugrohr (20) mit entweder geradem oder gebogenem Profil, wobei das Absaugrohr einen vergrößerten Mittenbereich (23), einen Vorderbereich (24), der sich nach vorn vom Mittenbereich aus erstreckt, eine Spitze in dem Ende des vorderen Bereichs, entgegengesetzt dem Mittenbereich aufweist, wobei der Vorderbereich im Durchmesser geringer ist als der vergrößerte Mittenbereich, wobei die Hülse einen länglichen, rohrförmigen Körper (12) aufweist, der einen Innenkanal (19) mit einem offenen, rückwärtigen Absaugende (15) und einem Vorderspitzenende (16) aufweist, wobei der rohrförmige Körper deformierbar ist, um gleitend mit dem Vorderbereich des chirurgischen Absaugrohres in Eingriff zu kommen und dessen Profil anzunehmen, wobei der Körper mehrere, beabstandete Öffnungen (18) zumindest im vorderen Spitzenbereich aufweist, die vom Spitzenende nach hinten beabstandet sind, um über den rohrförmigen Körper zu kommunizieren und den Durchgang des anzusaugenden Materials in den durch den rohrförmigen Körper definierten Innenkanal (19) zu ermöglichen,
dadurch **gekennzeichnet,** daß das Spitzenende der Hülse geschlossen ist und der Kanal des rohrförmigen Körpers ausgebildet ist zum lösbaren Aufnehmen des Vorderbereichs des Absaugrohres und worin der Vorderbereich und die Spitze des Absaugrohres sicher aber lösbar darin aufgenommen sind und daß das offene, rückwärtige Ende des rohrförmigen Körpers einen elastisch expandierbaren Abschnitt aufweist, der sich deutlich in der Größe ausdehnen kann, um reibend um den vergrößerten Mittenbereich des Absaugrohres in Eingriff zu kommen.

2. Hülse und Absaugrohrkombination nach Anspruch 1,
wobei die Innenfläche der Hülse (14) eine Vielzahl von nach innen vorstehenden Elementen (17) definiert, um das eingesetzte Absaugrohr in einer Mittenposition innerhalb des Kanals (19), der durch die Hülse definiert ist, in Position zu halten.

3. Hülse und Absaugrohrkombination nach Anspruch 1 oder 2,
wobei die Außenfläche der Hülse (13) eine Vielzahl von sich nach außen erstreckenden Rippen (31) aufweist, um das Beabstanden der Öffnungen (18), die in der Hülse vorgesehen sind, von Gewebe, das einen Operationsort bildet, zu unterstützen.

4. Hülse und Absaugrohrkombination nach einem der Ansprüche 1 bis 3,
aus elastisch deformierbarem Material.

5. Hülse und Absaugrohrkombination nach einem der Ansprüche 1 bis 4,
wobei die Größe des Innenkanals (19) des rohrförmigen Körpers (12) ausreichend größer ist als das äußere des Vorderbereichs des Absaugrohres (20), um zu ermöglichen, das Material, welches durch den Innenkanal (19) durch die Öffnungen (18) gelangt, durch den Innenkanal (19) zwischen dem Inneren des rohrförmigen Körpers (12) und dem äußeren des Absaugrohres (20) in Richtung auf die Vorderspitze des Absaugrohres (20) fließt.

6. Hülse und Absaugrohrkombination nach einem der vorstehenden Ansprüche,
wobei die Öffnungen (18) in radial beabstandeten, sich axial erstreckenden Linien angeordnet sind und wobei die inneren und die äußeren Abstandsrippen (17, 31) radial koinzident sind und sich zwischen den Linien beabstandeter Öffnungen erstrecken.

## Revendications

1. Manchon en combinaison avec un tube allongé (20) d'aspirateur chirurgical ayant un profil droit ou courbe, le tube d'aspirateur ayant une partie centrale élargie (23), une partie avant (24) s'étendant en avant de la partie centrale, un embout a' l'extémité de la partie avant opposé à la partie centrale, la partie avant ayant un diamètre inférieur à celui de la partie centrale élargie ; ledit embout comprenant un corps tubulaire allongé (12) définissant un canal interne (19) ayant une extrémité (15) d'aspirateur arrière ouverte et une extrémité avant d'embout (16), ledit corps tubulaire étant déformable pour s'engager de façon coulissante sur et adopter le profil de la partie avant du tube d'aspirateur chirurgical ; ledit corps définissant plusieurs orifices espacés (18) au moins dans la partie avant de l'embout en éloignement vers l'arrière de l'extrémité de l'embout, pour communiquer à travers le corps tubulaire pour permettre le passage de matériau devant être aspiré dans le canal interne (19) défini par le corps tubulaire, caractérisé en ce que l'extrémité de l'embout du manchon est fermée et le canal dudit corps tubulaire est réalisé pour y recevoir de façon amovible la partie avant du tube d'aspirateur et dans lequel la partie avant et l'embout du tube d'aspirateur y sont reçus de façon fixe mais amovible ; et en ce que l'extrémité arrière ouverte du corps tubulaire a une section résiliente expansible qui est capable de se dilater significativement en taille pour s'engager par friction autour de la partie centrale élargie du tube d'aspirateur.

2. Combinaison de manchon et de tube d'aspirateur selon la revendication 1, dans lequel la surface interne (14) du manchon définit une pluralité d'éléments (17) saillants vers l'intérieur pour maintenir le tube d'aspirateur en position lorsqu'il est utilisé dans une position centrale à l'intérieur du canal (19) défini par le manchon.

3. Combinaison de manchon et de tube d'aspirateur selon la revendication 1 ou la revendication 2, dans lequel la surface externe (13) du manchon présente une pluralité de nervures (31) saillantes vers l'extérieur pour contribuer à séparer les orifices (18) définis dans le manchon des tissus définissant un site chirurgical.

4. Combinaison de manchon et de tube d'aspirateur selon l'une des revendications 1 à 3, formée à partir d'un matériau résilient déformable.

5. Combinaison de manchon et de tube d'aspirateur selon l'une quelconque des revendications 1 à 4, dans lequel la taille du canal interne (19) du corps tubulaire (12) est suffisamment plus large que l'extérieur de la partie avant du tube (20) d'aspirateur pour permettre au matériau passant dans le canal intere (19) au travers des orifices (18) de s'écouler à travers le canal interne (19) entre l'intérieur du corps tubulaire (12) et l'extérieur du tube d'aspirateur (20) vers l'embout avant du tube d'aspirateur (20).

6. Combinaison de manchon et de tube d'aspirateur selon l'une quelconque des revendications précédentes, dans lequel les orifices (18) sont disposés suivant des lignes espacées radialement s'étendant axialement et les nervures d'espacement interne et externe (17,31) coincident radialement et s'étendent entre les lignes d'orifices espacées.
